# EUROPÄISCHE PATENTANMELDUNG

(11) **EP 3 384 958 A1**
(43) Veröffentlichungstag der Anmeldung: **10.10.2018**
(21) Anmeldenummer: 17164490.9
(22) Anmeldetag: 03.04.2017
(51) Int. Cl.: A61N 1/375

(54) **DURCHFÜHRUNG FÜR IMPLANTATSKOMPONENTEN**

(71) Anmelder: BIOTRONIK SE & Co. KG, 12359 Berlin (DE)
(72) Erfinder: Eigenbrod, Hella, 90530 Wendelstein (DE)
(74) Vertreter: Keck, Hans-Georg

(57) **Zusammenfassung**

Durchführung eines implantierbaren medizinelektronischen Gerätes, welches elektrische und/oder elektronische Funktionseinheiten umfasst, zum externen Anschluss mindestens einer der Funktionseinheiten, aufweisend: eine Mehrzahl von leitenden Anschlusselementen , die in gleich großen Gruppen in mindestens zwei Keramik-Isolierkörpern fixiert sind, indem sie vorab erzeugte, an die Form der Anschlusselemente angepasste Öffnungen der Isolierkörper durchstoßen und in diesen stoffschlüssig befestigt sind, und einen Durchführungs-Flansch, mit an die Außengestalt der Keramik-Isolierkörper angepassten Öffnungen, in denen die Keramik-Isolierkörper stoffschlüssig befestigt sind und der seinerseits zum Einschweißen in eine Geräteöffhung des Gerätes und damit zur Anbringung der Anschlusselemente im Gerät derart ausgebildet ist, dass deren geräteinnere Enden in vorbestimmten Positionen platziert sind, wobei die Keramik-Isolierkörper jeweils zwei zueinander parallele und identisch gestaltete Hauptoberflächen haben und zueinander rotationssymetrisch um eine senkrecht zu ihren Hauptoberflächen gerichtete Achse geformt sind.

## Beschreibung

Die Erfindung betrifft eine Durchführung eines implantierbaren medizinelektronischen Gerätes, welches elektrische und/oder elektronische Funktionseinheiten umfasst, zum externen Anschluss mindestens einer der Funktionseinheiten. Sie betrifft des Weiteren ein medizinelektronisches Gerät, welches eine solche Durchführung enthält.

Implantierbare Geräte der oben bezeichneten Art sind insbesondere als Herzschrittmacher, implantierbare Kardioverter (speziell Defibrillatoren) oder auch als Cochlearimplantate seit langem in massenhaftem Einsatz. Jedoch kann es sich auch um Neurostimulatoren oder Retinaimplantate oder ähnliche Geräte zur Beeinflussung der Sinneswahrnehmung bzw. Gehirn- und Nerventätigkeit handeln.

Die meisten praktisch bedeutsamen implantierbaren medizinelektronischen Geräte sind dazu vorgesehen, über geeignet platzierte Elektroden elektrische Impulse an reizbares Körpergewebe abzugeben oder elektrische Signale des Körpers zu messen. Um diese Funktion auszuführen, sind im Gehäuse des Gerätes elektronische/elektrische Funktionseinheiten zur Verarbeitung der Impulse und/oder zur geeigneten Steuerung der Reizerzeugung untergebracht, und außen am Gerät sind unmittelbar Elektroden oder Anschlüsse für mindestens eine Elektrodenleitung vorgesehen, in deren distalem Endabschnitt die Elektroden zur Impulsübertragung an das Gewebe angebracht sind.

Hierfür muss eine elektrische Verbindung zwischen den im Gehäuseinneren angeordneten elektrischen und/oder elektronischen Bauteilen und der jeweiligen Elektrodenleitung bzw. den extern angeordneten Elektroden und/oder Sensoren hergestellt werden. Diese elektrische Verbindung wird in der Regel mittels einer Durchführung und/oder einem sogenannten Header realisiert.

Eine derartige Durchführung sorgt dabei für mindestens eine elektrische Verbindung zwischen dem Inneren des Gehäuses und dem Äußeren und schließt gleichzeitig das Gehäuse des Implantats hermetisch ab. Der über der Durchführung befestigte Header führt die elektrische Verbindung der Durchführung weiter zu einer Kontaktstelle und dient zum Einstecken der mindestens einen Elektrodenleitung in eine entsprechende, meist genormte Buchse. An den Kontaktstellen der Buchse wird so ein elektrischer Kontakt zwischen dem Implantat und dem Anschlussstück der Elektrodenleitung hergestellt. Eine Durchführung und ein Header können auch in einem einzigen Bauteil realisiert sein. Auch in diesem Fall wird ein solches kombiniertes Bauteil im Folgenden allgemein als Durchführung bezeichnet.

Bei an sich bekannten Durchführungen, die zwei in ihrer Grundform flach prismatische Keramik-Isolierkörper (mit abgerundeten Kantenbereichen) umfassen, sind die Durchgangsöffnungen, die in den Isolierkörpern für die Anschlussstifte geschaffen sind, auf einer Hauptoberfläche des Isolierkörpers mit einem sich trichterförmig absenkenden Randbereich, der sog. ,Einführschräge', versehen. Diese Absenkung dient als kleines Reservoir für das Hartlot und soll auch verhindern, dass dieses die Oberfläche des Isolierkörpers zu weit übersteigt und dann in Kontakt mit einem Material nahe dem oberen Ende des Anschlussstiftes, welches als Träger für ein Weichlot geeignet ist, oder mit dem dort später aufgebrachten Weichlot kommen könnte. Bei einem solchen Kontakt entstehende Mischphasen könnten zu Materialversprödungen und Brüchen und letztlich zu Zuverlässigkeitsproblemen bei dem medizinelektronischen Gerät führen.

In Folge dieser trichterförmigen Absenkungen auf der einen Hauptoberfläche der Keramik-Isolierkörper müssen, je nach deren Grundform, verschiedene Ausführungen erzeugt und bei der Bestückung der Durchführung auch voneinander unterschieden und korrekt in den Durchführungs-Flansch eingesetzt werden, was einen entsprechenden herstellungstechnischen und logistischen Aufwand erfordert und die Ausschuss-Gefahr erhöht.

Der Erfindung liegt die Aufgabe zugrunde, eine Durchführung mit an sich bekanntem und bewährtem Aufbau anzugeben, die mit verringertem logistischen und herstellungstechnischen Aufwand zu erzeugen ist und dennoch eine hohe Langzeit-Zuverlässigkeit hat. Diese Aufgabe wird durch eine Durchführung mit den Merkmalen des Anspruchs 1 gelöst. Zweckmäßige Fortbildungen des Erfindungsgedankens sind Gegenstand der abhängigen Ansprüche. Es wird weiterhin ein verbessertes implantierbares medizinelektronisches Gerät mit den Merkmalen des Anspruchs 10 bereitgestellt.

Die Erfindung schließt den Gedanken ein, eine Durchführung mit mehreren Keramik-Isolierkörpern zu bestücken, die in ihrer Grundform wie auch der Gestaltung der beiden Hauptoberflächen, d. h. derjenigen Oberflächen, aus denen die Anschlusselemente beidseits herausragen, identisch sind. Dies erfordert insbesondere eine Abkehr vom etablierten Stand der Technik insofern, als auf die o. a. Einführschrägen zu verzichten ist. Die in den Augen des Fachmanns mit einem solchen Abgehen von der üblichen Gestaltung verbundenen Nachteile sind bis zu einem gewissen Grade in Kauf zu nehmen; die Isolierkörper sind aber insgesamt so auszuführen, dass sich hierdurch keine Probleme bei der Langzeit-Zuverlässigkeit ergeben.

In aus derzeitiger Sicht bevorzugten Ausführungen haben die Keramik-Isolierkörper die Grundform eines flachen Prismas mit der Grundfläche eines Parallelogramms mit abgerundeten Ecken. Mit dieser Grundform sind die Isolierkörper unproblematisch in etablierte Handling- und Bestückungsabläufe der Durchführungen von Herzschrittmachern und ähnlichen Geräten integrierbar.

In einer weiteren aktuell bevorzugten Ausführung hat die Durchführung insgesamt 12 in zwei oder drei Keramik-Isolierkörpern mit jeweils gleicher Anzahl von Anschlusselementen angeordnete Anschlusselemente. Wenn insbesondere zwei Isolierkörper vorgesehen sind, sind in jedem 6 Anschlusselemente angeordnet, beispielsweise in zwei Reihen nahe den langen Kanten der Isolierkörper.

In weiteren Ausführungen der Erfindung ist ein Masse-Anschlusselement zwischen den Keramik-Isolierkörpern angeordnet. Hierbei handelt es sich wiederum um eine Ausführung, die - insbesondere in Kombination mit zwei prismatischen Isolierkörpern mit Parallelogramm-Grundfläche - mit bewährten Herstellungstechnologien leicht zu realisieren ist.

In weiteren Ausführungen der Erfindung sind die Keramik-Isolierkörper mindestens auf einer Hauptoberfläche mit einer die Hartlötfähigkeit verbessernden Dünnschicht versehen. Viele für den Einsatzzweck grundsätzlich geeignete Keramiken haben eine ungenügende oder jedenfalls den Anforderungen nicht in vollem Umfang genügende Benetzbarkeit bezüglich typischer Hartlote (etwa Gold oder Goldlegierungen), so dass der zusätzliche Aufwand eines Beschichtungsschrittes sich im Interesse einer hohen Produktqualität lohnt.

In praxisrelevanten Ausführungen der Erfindung ist als Verbindungsmittel zur stoffschlüssigen Befestigung der Anschlusselemente in den Keramik-Isolierkörpern und der Keramik-Isolierkörper im Durchführungs-Flansch jeweils ein Hartlot, insbesondere Gold oder eine Gold-Legierung, vorgesehen.

In einer weiteren bevorzugten Ausführung der Erfindung sind beide Hauptoberflächen der Keramik-Isolierkörper, einschließlich Randbereichen der von den Anschlusselementen durchstoßenen Öffnungen, eben ausgebildet. Hiervon ausgenommen sind optional die äußeren Randbereiche der Isolierkörper, die insbesondere abgeschrägt oder auch abgerundet sein können, um das Vorhandensein beschädigungsempfindlicher Außenkanten zu vermeiden.

Insbesondere das Fortlassen bei vergleichbaren bekannten Isolierkörpern vorgesehenen trichterförmigen Absenkungen um die Öffnungen für die Anschlusselemente beruht auf einer Abkehr von fest verankerten Vorstellungen. Hiernach waren solche Absenkungen für eine hohe Produktqualität der bestückten Durchführung wesentlich. Untersuchungen der Erfinderin haben aber gezeigt, dass eine allen Anforderungen genügende Konfiguration des Hartlotes bezüglich des Isolierkörpers einerseits und der Anschlusselemente anderseits auch ohne diese sog. Einführschrägen zu erreichen ist. Deren Fortlassen führt nämlich weder zu einer verschlechterten Fixierung der Anschlusselemente im Isolierkörper noch zu einem unzulässigen "Aufsteigen" des Hartlotes am Anschlusselement in Richtung auf den Nailhead.

Weiterhin sind insbesondere die Anschlusselemente als Anschlussstifte in Nailhead-Form derart ausgeführt, dass sie einen zylindrischen Stiftkörper und mindestens einseitig einen aufgesetzten Nailhead mit vergrößertem Durchmesser umfassen. Hierbei weist der Stiftkörper mindestens auf der Umfangsfläche ein Material mit guter Hartlötfähigkeit auf, und der Nailhead ist aus einem Material mit guter Weichlötfähigkeit gebildet. Bei letzterem Material kann es sich um Cu, Ni, Ag, Sn, Au, Pt, Ir, Pd oder Legierungen aus mindestens zweien dieser oder ein die Weichlötbarkeit verbessernden Schichtsystem, wie etwa EPIG, ENEPIG, HAL oder dergleichen handeln.

Der "stumpf" aufgesetzte Nailhead hat gegenüber etablierten Alternativlösungen den Vorteil, dass der Abstand zwischen der Unterseite des Nailhead und der Oberfläche des Isolierkörpers präzise definiert und relativ groß gehalten werden kann, was dabei hilft, das oben als nachteilig erwähnte Zusammentreffen von Hartlot und Weichlot bei der fertig verlöteten Durchführung zu verhindern.

In diesem Sinne ist bei bevorzugten Ausführungen der Erfindung vorgesehen, dass das Hartlot einen Teil der Umfangsfläche der Stiftkörper über derjenigen Hauptoberfläche der Keramik-Isolierkörper benetzt, über denen die Nailheads der Anschlussstifte vorgesehen sind, dass die Hartlot-Benetzung aber mit einem definierten Abstand unter der Unterseite der Nailheads endet.

Vorteile und Zweckmäßigkeiten der Erfindung ergeben sich im Übrigen aus der nachfolgenden Beschreibung eines Ausführungsbeispiels anhand der Figuren. Von diesen zeigen:
- Fig. 1: eine schematische, teilweise geschnittene Darstellung eines implantierbaren medizinelektronischen Geräts und
- Fig. 2A - 2C: eine perspektivische Darstellung, schematische Draufsicht und schematische Seitenansicht einer Ausführungsform der erfindungsgemäßen Durchführung.

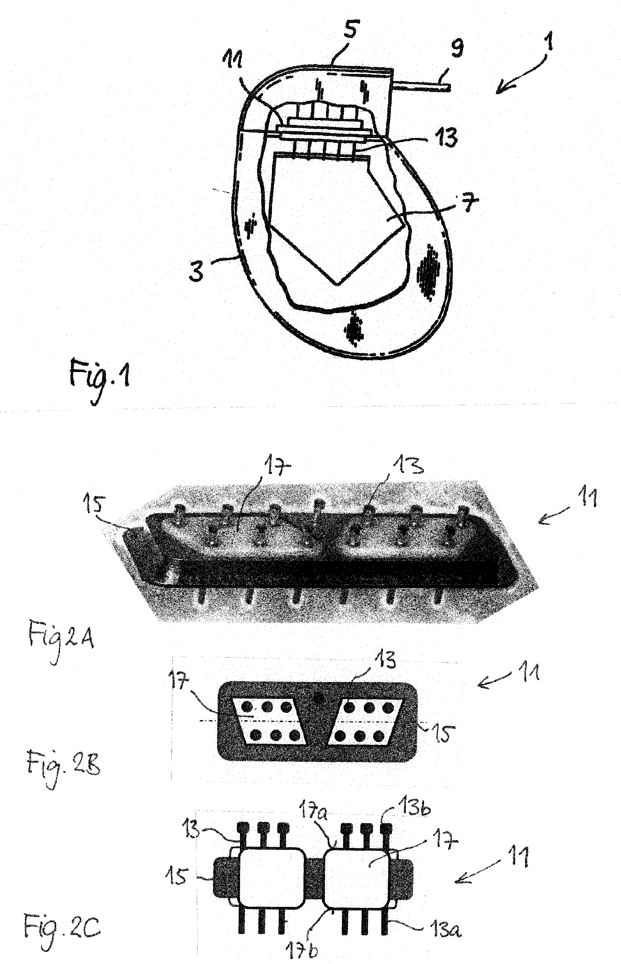

Fig. 1 zeigt einen Herzschrittmacher 1 mit einem Schrittmachergehäuse 3 und einem Kopfteil (Header) 5, in dessen Innerem neben anderen elektronischen Komponenten eine Leiterplatte (PCB) 7 angeordnet und mit dessen im Header angeordneten (nicht gezeigten) Leitungsanschluss eine Elektrodenleitung 9 verbunden ist. Eine zwischen Gerätegehäuse 3 und Header 5 vorgesehene Durchführung 11, die in einem Durchführungs-Flansch 12 gehaltert ist, umfasst eine Mehrzahl von Anschlusselementen 13 zum externen Anschluss der Leiterplatte 7.

Fig. 2A - 2C zeigen eine Ausführungsform der Durchführung 11 mit einem langgestreckt rechteckigen Durchführungs-Flansch 15, beispielsweise aus Titanblech geformt, und zwei hierin eingesetzten Keramik-Isolierkörpern 17, in die jeweils sechs Anschlussstifte 13 eingesetzt sind.

Die Isolierkörper 17 haben die Gestalt eines Prismas mit der Grundfläche eines Parallelogramms mit allseits abgerundeten oder abgeschrägten Kantenbereichen und Hauptoberflächen 17a, 17b. Die Isolierkörper sind aus einer der für diese Anwendung bekannten Keramiken, etwa AI₂O₃, und mit bekannten Technologien gefertigt, so dass diesbezüglich eine genauere Beschreibung nicht erforderlich ist. Die Hauptoberflächen 17a, 17b, sind bis unmittelbar an den Rand der (nicht gesondert bezeichneten) Öffnungen für die Anschlussstifte 13 mit Ausnahme der Kantenbereiche, völlig eben. Aufgrund dessen sind die beiden Keramik-Isolierkörper 17 identisch, so dass nur ein Isolierkörper-Typ gefertigt und bei der Bestückung der Durchführung 11 eingesetzt werden muss.

Die Anschlussstifte 13 haben, wie in Fig. 2a und 2c zu erkennen ist, einen Nailhead-Aufbau mit einem zylindrischen Stiftkörper 13a und einem stumpf aufgesetzten Nailhead 13b mit vergrößertem Durchmesser. Der Stiftkörper 13a besteht aus einem für derartige Bauelemente bekannten Material, etwa Niob, Platin, Iridium, einer Platin/Iridium-Legierung, Tantal, Titan, Zirkonium, Hafnium oder Legierungen aus diesen Materialien oder einem medizinischen Edelstahl. Der Nailhead besteht aus einem gut weichlötbaren Material, wie einem der weiter oben genannten. Die Anschlussstifte 13 sind in die Isolierkörper 17 ebenso mit einem Hartlot auf Gold-Basis eingelötet, wie die Isolierkörper 17 in den Durchführungs-Flansch 15.

Zwischen den Keramik-Isolierkörpern 17 ist in den Durchführungs-Flansch 15, mit diesem leitend verbunden, ein Masse-Anschlussstift 13' eingelötet. Der Masse-Anschlussstift hat im Wesentlichen den gleichen Aufbau wie die in die Isolierkörper 17 eingefügten Anschlussstifte 13.

Im Übrigen ist die Ausführung der Erfindung auch in einer Vielzahl von Abwandlungen der hier gezeigten Beispiele und weiter oben hervorgehobenen Aspekte der Erfindung möglich.

## Patentansprüche

1. Durchführung (11) eines implantierbaren medizinelektronischen Gerätes (1), welches elektrische und/oder elektronische Funktionseinheiten (7) umfasst, zum externen Anschluss mindestens einer der Funktionseinheiten, aufweisend:
eine Mehrzahl von leitenden Anschlusselementen (13), die in gleich großen Gruppen in mindestens zwei Keramik-Isolierkörpern (17) fixiert sind, indem sie vorab erzeugte, an die Form der Anschlusselemente angepasste Öffnungen der Isolierkörper durchstoßen und in diesen stoffschlüssig befestigt sind, und
einen Durchführungs-Flansch (15), mit an die Außengestalt der Keramik-Isolierkörper angepassten Öffnungen, in denen die Keramik-Isolierkörper stoffschlüssig befestigt sind und der seinerseits zum Einschweißen in eine Geräteöffnung des Gerätes und damit zur Anbringung der Anschlusselemente im Gerät derart ausgebildet ist, dass deren geräteinnere Enden in vorbestimmten Positionen platziert sind,
wobei die Keramik-Isolierkörper jeweils zwei zueinander parallele und identisch gestaltete Hauptoberflächen (17a, 17b) haben und zueinander rotationssymetrisch um eine senkrecht zu ihren Hauptoberflächen gerichtete Achse geformt sind.

2. Durchführung nach Anspruch 1, wobei die Keramik-Isolierkörper (17) die Grundform eines Prismas mit der Grundfläche eines Parallelogramms mit abgerundeten Ecken haben.

3. Durchführung nach Anspruch 1 oder 2, mit insgesamt 12 in zwei oder drei Keramik-Isolierkörpern (17) mit jeweils gleicher Anzahl von Anschlusselementen angeordneten Anschlusselementen (13).

4. Durchführung nach Anspruch 3, wobei ein Masse-Anschlusselement (13') zwischen den Keramik-Isolierkörpern (17) angeordnet ist.

5. Durchführung nach einem der vorangehenden Ansprüche, wobei die Keramik-Isolierkörper (17) mindestens auf einer Hauptoberfläche (17a) mit einer die Hartlötfähigkeit verbessernden Dünnschicht versehen sind.

6. Durchführung nach einem der vorangehenden Ansprüche, wobei beide Hauptoberflächen (17a, 17b) der Keramik-Isolierkörper (17), einschließlich Randbereichen der von den Anschlusselementen durchstoßenen Öffnungen, aber optional mit Ausnahme der äußeren Randbereiche, eben ausgebildet sind.

7. Durchführung nach einem der vorangehenden Ansprüche, wobei als Verbindungsmittel zur stoffschlüssigen Befestigung der Anschlusselemente (13) in den Keramik-Isolierkörpern (17) und der Keramik-Isolierkörper im Durchführungs-Flansch (15) jeweils ein Hartlot, insbesondere Gold oder eine Gold-Legierung, vorgesehen ist.

8. Durchführung nach Anspruch 7, wobei die Anschlusselemente als Anschlussstifte (13) in Nailhead-Form derart ausgeführt sind, dass sie einen zylindrischen Stiftkörper (13a) und mindestens einseitig einen aufgesetzten Nailhead (13b) mit vergrößertem Durchmesser umfassen, wobei der Stiftkörper mindestens auf der Umfangsfläche ein Material mit guter Hartlötfähigkeit aufweist und der Nailhead aus einem Material mit guter Weichlötfähigkeit gebildet ist.

9. Durchführung nach Anspruch 7 und Anspruch 8, wobei das Hartlot einen Teil der Umfangsfläche der Stiftkörper (13a) über derjenigen Hauptoberfläche (17a) der Keramik-Isolierkörper (17) benetzt, über denen die Nailheads (13b) der Anschlussstifte (13) vorgesehen sind, wobei die Hartlot-Benetzung mit einem Abstand unter der Unterseite der Nailheads endet.

10. Implantierbares medizinelektronisches Gerät (1), insbesondere Herzschrittmacher, Cardioverter oder Cochlearimplantat, mit einer Durchführung (11) nach einem der vorangehenden Ansprüche, die in einer Geräteöffnung des Gerätes mit einer Laserschweißnaht befestigt sind.
